# EUROPEAN PATENT APPLICATION

(11) **EP 4 624 442 A1**
(43) Date of publication of application: **01.10.2025**
(21) Application number: 23894653.7
(22) Date of filing: 22.11.2023
(51) Int. Cl.: C07C 1/02, B01J 23/83, C07B 61/00, C07C 9/04, C10G 2/00

(54) **CATALYST FOR FISCHER-TROPSCH SYNTHESIS REACTION AND HYDROCARBON PRODUCTION DEVICE**

(30) Priority: 22.11.2022 JP 2022186945
(71) Applicant: Sumitomo Heavy Industries, Ltd., Tokyo 141-6025 (JP)
(72) Inventor: SUZUKI, Takashi, Yokosuka-shi, Kanagawa 237-8555 (JP)
(74) Representative: Louis Pöhlau Lohrentz
(86) International application number: PCT/JP2023/042072
(87) International publication number: WO 2024/111649

(57) **Abstract**

According to an aspect of the present invention, there is provided a production apparatus including an FT reaction catalyst which can efficiently convert carbon dioxide into a hydrocarbon in an FT reaction of a raw material gas containing carbon dioxide.

In order to achieve the aspect of the present invention, there is provided a hydrocarbon production apparatus. The hydrocarbon production apparatus supplies a raw material gas into a reaction vessel having a reaction catalyst to produce a hydrocarbon by an FT synthesis reaction. The raw material gas contains carbon dioxide, and the reaction catalyst contains at least one element selected from yttrium, cerium, lanthanum, praseodymium, neodymium, and holmium. Since the hydrocarbon production apparatus is used and further a combination of an optimal catalyst composition, a proper pretreatment or a holding amount of a catalyst, a suitable reaction condition, and the like is selected, efficient conversion of not only carbon monoxide but also carbon dioxide into a hydrocarbon can be achieved.

## Description

### Technical Field

The present invention relates to a Fischer-Tropsch synthesis (hereinafter, referred to as FT synthesis). The FT synthesis is a reaction of causing a mixed gas of carbon monoxide and hydrogen to react in the presence of a catalyst to produce a hydrocarbon mixture.

### Background Art

The FT synthesis reaction is a gas-solid contact reaction in which a synthesis gas is reacted by using a solid catalyst, and a fixed bed type reactor is initially used as a reaction apparatus thereof. In addition, there is a fluidized bed reactor such as a slurry type or a circulating fluidized type.

According to PTL 1, a catalyst composition containing iron, cobalt, and ruthenium as main catalysts, and further containing a non-zeolite type silicon-aluminophosphate molecular sieve as an auxiliary catalyst/carrier is used. In this manner, selectivity and a conversion rate of a liquid hydrocarbon having a gasoline equivalent fraction from a hydrocarbon having 5 carbon atoms (hereinafter, referred to as C5) to 420°F (216°C) are improved.

In addition, PTL 2 discloses an example as follows. A manganese oxide carrier to which an alkali metal (potassium in an example) is added is used for a ruthenium-based catalyst, which is originally and mainly used in a fixed bed type reactor. In this manner, a liquid phase slurry method is applied to improve olefin selectivity in an olefin/paraffin ratio and a conversion rate of a hydrocarbon to be produced.

In addition, in the FT synthesis reaction, the following fact is known. In a case where carbon dioxide is used as the raw material gas in addition to carbon monoxide, when a proportion of carbon dioxide increases, conversion is unlikely to occur from the carbon dioxide to the hydrocarbon, and a raw material conversion transfer rate on a C base is reduced. On the other hand, generation of methane increases.

### Citation List

### Patent Literatures

[PTL 1] PCT Japanese Translation Patent Publication No. H61-502897
[PTL 2] Japanese Unexamined Patent Publication No. 2002-161279

### Summary of Invention

### Technical Problem

However, when the carbon dioxide is contained in the raw material gas, there is a problem in that a study on a technique for efficiently converting carbon atoms thereof into the hydrocarbon or prominent means therefor is not yet sufficient.

Therefore, the present invention aims to provide a production apparatus including an FT reaction catalyst which can efficiently convert carbon dioxide into a hydrocarbon in an FT reaction of a raw material gas containing carbon dioxide.

### Solution to Problem

The present inventor has intensively studied the above-described problems. As a result, the present invention is completed by finding that carbon dioxide can be efficiently converted into a hydrocarbon by selecting a catalyst for a proper FT synthesis reaction.

That is, the present invention provides a catalyst for an FT synthesis reaction, a hydrocarbon production apparatus, and a hydrocarbon production method according to the following aspects.

According to the present invention, in order to solve the above-described problems, there is provided a hydrocarbon production apparatus. The hydrocarbon production apparatus supplies a raw material gas into a reaction vessel having a reaction catalyst to produce a hydrocarbon by an FT synthesis reaction. The raw material gas contains carbon dioxide, and the reaction catalyst contains at least one element selected from yttrium, cerium, lanthanum, praseodymium, neodymium, holmium, sodium, potassium, rubidium, cesium, beryllium, magnesium, calcium, strontium, barium, and copper.

According to this aspect, a combination of an optimal catalyst composition, a proper pretreatment or a holding amount of a catalyst, a suitable reaction condition, and the like is selected. In this manner, efficient conversion of not only carbon monoxide but also carbon dioxide into a hydrocarbon can be achieved.

In addition to the above-described aspect, as one embodiment of the hydrocarbon production apparatus of the present invention, the reaction catalyst contains at least one element selected from cobalt, ruthenium, and iron.

According to this aspect, a conversion rate of the carbon into the hydrocarbon can be improved, and an efficient FT reaction can be performed.

In addition to the above-described aspect, as one embodiment of the hydrocarbon production apparatus of the present invention, a holding amount of the catalyst inside the reaction vessel is 2% by mass or more and 25% by mass or less with respect to a weight of an organic medium inside the reaction vessel.

According to this aspect, the conversion rate of the carbon in the raw material gas into the hydrocarbon can be properly improved, and the efficient FT reaction can be performed.

In addition to the above-described aspect, as one embodiment of the hydrocarbon production apparatus of the present invention, the raw material gas contains carbon monoxide, and a proportion of the carbon dioxide to a total amount of the carbon monoxide and the carbon dioxide is 1% by volume or higher.

According to this aspect, the carbon dioxide is usefully used. The present invention can contribute to carbon recycling which has been a problem in recent years, and can also be used for coping with environmental problems.

In addition, as one embodiment of the hydrocarbon production apparatus of the present invention, the hydrocarbon production apparatus further includes a plate-shaped heat exchange unit having irregularities on a surface.

According to this aspect, heat generated by the FT reaction inside the reactor can be efficiently removed, and a reaction temperature can be accurately controlled.

According to the present invention, in order to solve the above-described problems, there is provided a hydrocarbon production method. In the hydrocarbon production method, a raw material gas is supplied into a reaction vessel having a reaction catalyst to produce a hydrocarbon by a Fischer-Tropsch synthesis reaction. The raw material gas contains carbon dioxide, and the reaction catalyst contains at least one element selected from yttrium, cerium, lanthanum, praseodymium, neodymium, holmium, sodium, potassium, rubidium, cesium, beryllium, magnesium, calcium, strontium, barium, and copper.

According to this aspect, a combination of an optimal catalyst composition, a proper pretreatment or a holding amount of a catalyst, a suitable reaction condition, and the like is selected. In this manner, efficient conversion of not only carbon monoxide but also carbon dioxide into a hydrocarbon can be achieved.

### Advantageous Effects of Invention

According to the present invention, it is possible to provide a production apparatus including an FT reaction catalyst which can efficiently convert carbon dioxide into a hydrocarbon in an FT reaction of a raw material gas containing carbon dioxide.

### Brief Description of Drawings

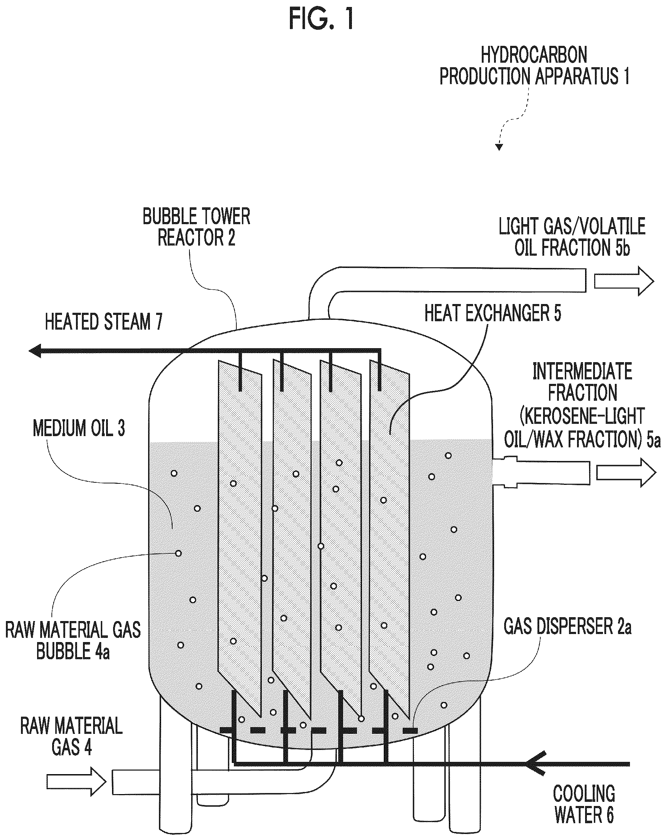

Fig. 1 is a schematic view for describing a hydrocarbon production apparatus of the present invention.
Fig. 2 is a graph showing an experimental result of an example.
Fig. 3 is a schematic view for describing a fixed bed type reactor.

### Description of Embodiments

The present invention relates to an FT reaction apparatus that efficiently performs an FT reaction by appropriately selecting a reaction catalyst in a hydrocarbon production apparatus using an FT synthesis reaction and accurately controlling heat generation during a reaction.

Hereinafter, a catalyst for the FT synthesis reaction and the hydrocarbon production apparatus according to the present invention will be described in detail with reference to the drawings.

The catalyst for the FT synthesis reaction and the hydrocarbon production apparatus which are described in the embodiment are merely examples for describing the hydrocarbon production apparatus according to the present invention, and the present invention is not limited thereto. In addition, a hydrocarbon production method will be replaced in the hydrocarbon production apparatus.

### [First Embodiment]

A hydrocarbon production apparatus 1 of a first embodiment is an apparatus configured as follows. A raw material gas is blown into a medium oil in which a fine powder-shaped catalyst for an FT reaction is suspended to react the raw material gas. The raw material gas is converted into a hydrocarbon fraction to extract the hydrocarbon fraction. In addition, the hydrocarbon production apparatus 1 includes a heat exchanger for removing reaction heat thereof and controlling a reaction temperature.

### [Reactor, Raw Material]

### <Reaction Vessel>

A reaction vessel of the present invention is a vessel internally containing a reaction catalyst and circulating the raw material gas to cause the FT reaction. Hereinafter, the present invention will be described by using a slurry type fluidized bed, but the present invention also includes a fixed bed type reactor using a gas-solid contact reaction. A fixed bed type has a high reaction yield. On the other hand, a slurry type has high temperature controllability, and is preferably used.

The reaction vessel of the first embodiment is a bubble tower reaction vessel 2, and is a pressure-resistant vessel to be used for the gas-liquid contact reaction. The reaction vessel is internally filled with a liquid, and a gas is blown from a lower portion, so that a reaction proceeds in a gas-liquid interface.

In the present invention, a medium oil 3 of a liquid hydrocarbon containing a fine powder-shaped catalyst is used as the liquid, and the reaction is caused to proceed by blowing and circulating a raw material synthesis gas in a fine foam shape from a bottom portion of a reactor. A gas disperser 2a that converts a raw material gas 4 into a minute raw material gas bubble 4a is installed in a lower portion of a bubble tower.

### <Medium Oil>

The medium oil 3 is a liquid medium for suspending a catalyst to fill the reaction vessel for the gas-liquid contact reaction. For example, the liquid hydrocarbon is used, but it is preferable to use a paraffin-based liquid hydrocarbon having 10 to 20 carbon atoms. The fine powder-shaped catalyst (to be described later) is suspended in the hydrocarbon.

The liquid hydrocarbon filling the reaction vessel in a reaction initial stage is substituted with a liquid hydrocarbon which is a product, as the reaction proceeds.

### <Raw Material Gas>

A raw material synthesis gas 4 is a raw material for producing a hydrocarbon by the FT reaction.

A raw material gas for a normal FT reaction contains hydrogen (H₂) and carbon monoxide (CO), but in the present invention, the raw material gas 4 contains carbon dioxide (CO₂). Since a rate of the FT reaction depends on a hydrogen partial pressure, a partial pressure of H₂ is required to a certain extent, and a partial pressure ratio (mole ratio) of the hydrogen of the raw material gas of the present invention to a total of (carbon monoxide + carbon dioxide) is suitably 0.6 to 2.7, preferably 0.8 to 2.5, and more preferably 1 to 2.3.

On the other hand, a ratio between carbon monoxide and carbon dioxide can be changed depending on a purpose thereof. For the purpose of carbon recycling, the ratio of carbon dioxide is increased, and in order to increase a conversion rate to the hydrocarbon, the ratio of the carbon monoxide is increased. In the present invention, the carbon monoxide may not be used in the carbon dioxide of 100%. The ratio of the carbon dioxide to the carbon monoxide when both are used is not particularly limited, but a proportion of the carbon dioxide to a total amount of the carbon monoxide and the carbon dioxide needs to be 1% by volume or higher. Preferably, the proportion is 10% or higher, more preferably 30% or higher, and still more preferably 40% or more.

As other components, for example, sulfur, organic nitrogen, phosphorus, and the like are clearly harmful, but substances other than the main components may be mixed as long as the substances do not hinder the reaction.

### <Product>

In a hydrocarbon mixture produced by the FT reaction, a light gas/volatile oil fraction 5b is taken out as a gas from an upper portion of the reactor. On the other hand, a light oil-kerosene fraction/wax fraction 5a having many chain carbon atoms is taken out from an upper portion of a liquid surface of the reactor, and is distilled and refined as appropriate.

### <Heat Exchanger>

A heat exchanger 5 is used to maintain a temperature inside the reaction vessel within a predetermined range.

Since the FT reaction is an exothermic reaction, the heat exchanger 5 having excellent heat exchange capability needs to be installed inside the reaction vessel. In the related art, a tubular or coil-shaped cooling pipe is generally used. However, the present invention adopts a heat exchanger having a structure in which many plate-shaped cooling plates having an irregular surface to increase a heat transfer surface area are densely stacked. Cooling water 6 introduced from a lower portion of the reactor passes through the cooling plates of the heat exchanger, and flows out from the upper portion of the reactor, as heated steam 7.

Cooling from the outside can be performed by a jacket outside the reactor in combination with the heat exchanger inside the reactor.

### [Reaction Conditions]

### <Reaction Pressure>

A chain growth of the FT reaction depends on a pressure, and in the present invention, the reaction is performed under a condition of 1000 kPa from a normal pressure, preferably 100 kPa or higher and 900 kPa or lower, more preferably 200 kPa or higher and 800 kPa or lower, and still more preferably 400 kPa or higher and 800 kPa or lower.

### <Reaction Temperature>

The FT reaction is generally performed at a temperature in a range of approximately 150°C to 300°C.

Here, when a reaction temperature of the catalyst 10 is lower than 200°C, the conversion rate of CO and CO₂ in the FT reaction tends to decrease, and when the reaction temperature is 270°C or higher, a distribution of the product is lightened, thereby causing is a possibility that a yield of an intermediate fraction is degraded.

Therefore, since the reaction temperature of the catalyst 10 of the present invention is set in a range of 200°C to 270°C, the carbon dioxide can be efficiently converted into a liquid hydrocarbon.

More preferably, the reaction temperature of the catalyst 10 is set in a range of 220°C to 250°C. Since the reaction temperature is maintained in this range, ability of the catalyst 10 can be maximized, and the carbon dioxide can be more efficiently converted into the liquid hydrocarbon.

### [Catalyst]

### <Main Catalyst>

As a main catalyst for the FT reaction, a type of metal catalyst selected from cobalt, ruthenium, and iron is used. Iron is inexpensive, but catalyst activity is relatively low, and the catalyst activity of ruthenium is high, but the ruthenium is very expensive noble metal. The product of the FT reaction caused by an iron-based catalyst has a feature of many naphtha fractions, and contains an oxygen-containing compound. Therefore, cobalt is most preferably used to obtain the intermediate fraction such as a light oil, a jet fuel, or a kerosene. Cobalt is also relatively expensive, and a research on a life of the catalyst and on increasing reactivity with a small amount of the catalyst has been conducted.

In the present invention, as the main catalyst, a catalyst containing one element selected from cobalt, ruthenium, and iron is used, and preferably, a catalyst containing cobalt is used.

In addition, the amount of the main catalyst (such as cobalt) is preferably 5% by weight or more and 25% by weight or less, as a weight-based carriage amount of main catalyst metal in the catalyst.

More preferably, the amount of the main catalyst is 7.5% by weight or more and 17% by weight or less, and still more preferably, 8% by weight or more and 15% by weight or less. When the carriage amount increases, the reaction is likely to proceed. However, when the carriage amount exceeds an upper limit of this range, the activity of the FT reaction with respect to an increase in the carriage amount tends to be saturated, and the configuration is no longer technically important in terms of cost. On the other hand, when the carriage amount is below a lower limit of this range, the activity per amount of the catalyst decreases. Therefore, this case is not preferable.

### <Catalyst Carrier>

As a catalyst carrier of the present invention, a carrier containing at least one type of silica (SiO₂), alumina (Al₂O₃), and zeolite (aluminosilicate) is used. Among these materials, it is preferable that the carrier contains silica. Since silica is chemically stable, silica is unlikely to affect the main catalyst or an auxiliary catalyst. Therefore, chemical characteristics of the main catalyst or the auxiliary catalyst can be sufficiently realized. In addition, since a specific surface area is large, contact efficiency with the raw material gas (substrate) is high, and the FT reaction can efficiently proceed. In addition, true density (true specific gravity) of the carrier is highest in the order of alumina, zeolite, and silica. Therefore, when dispersion of the catalyst inside the bubble tower reactor is considered, it is considered that silica has a most excellent operability among these materials.

### <Auxiliary Catalyst>

In the present invention, in addition to the main catalyst, the auxiliary catalyst contains at least one element selected from the group consisting of yttrium, cerium, lanthanum, praseodymium, neodymium, and holmium, which are rare earth elements, at least one element selected from the group consisting of sodium, potassium, rubidium, and cesium, which are alkali metal, at least one element selected from the group consisting of beryllium, magnesium, calcium, strontium, and barium, which are alkaline earth metal, and copper. Among these materials, yttrium, cerium, lanthanum, praseodymium, neodymium, holmium, and copper are preferable, yttrium, cerium, lanthanum, praseodymium, neodymium, and holmium are more preferable, and yttrium is most preferable.

It is considered that the auxiliary catalyst is added not only to increase an adsorption amount of the carbon monoxide and the carbon dioxide on a catalyst surface but also to increase the number of reaction active sites. In a case of copper, charge transfer occurs between the main catalyst (cobalt or the like) and copper, an oxidation number of the main catalyst slightly decreases (δ-), and an improved advantageous effect can be expected in a turnover frequency (TOF) at an active site of the FT reaction, which is one type of hydrogenation reactions of CO and CO₂.

As the amount of the auxiliary catalyst, it is preferable to contain the auxiliary catalyst of 1/30 to 1/3 of the weight-based carriage amount of the main catalyst (cobalt or the like). It is more preferable to contain 1/20 or more and 1/5 or less.

### <Catalyst Shape>

The reaction catalyst of the present invention has a fine powder shape, and preferably has a size of 0.07 mm or larger and 0.2 mm or smaller. More preferably, the size is 0.08 mm or larger and 0.17 mm or smaller. A particle size of the catalyst can be measured by a laser diffraction method. This method utilizes the following phenomenon. When particles are irradiated with laser beams, and the particles are scattered. A scattering angle increases in small particles, and decreases in large particles.

### <Preparation of Catalyst>

### <Determination of Saturated Water Absorption Amount>

The carrier material is heated or burned (sintered) in advance at 500°C or higher and 520°C or lower for 3 hours or longer under a dry air circulation. After the carrier material is cooled at a room temperature, highly pure water (hereinafter, pure water) such as ion-exchanged water is dropped to a predetermined amount of the carrier material until the water absorption is saturated with a burette or the like, and a water absorption amount per unit weight of the carrier material (ml (water)/g (carrier material)) is obtained. Hereinafter, the saturated water supply amount will be referred to as an ADF (adsorption factor). An upper limit of a sintering time is not particularly limited, but it may be considered that the upper limit is 6 hours from a viewpoint of productivity.

### <Carrying of Active Metal (Main Catalyst)>

The carrier material sintered at 500°C or higher and 520°C or lower for 3 hours or longer under the dry air circulation is cooled at the room temperature, and a predetermined amount of the carrier material is precisely weighed. A predetermined amount of the active metal raw material is dissolved by adding pure water thereto, and thereafter, water is added thereto to prepare an impregnation liquid until the active metal raw material has the amount of the ADF of the precisely weighed carrier material. The precisely weighed carrier material is immersed in the impregnation liquid, and is placed in a static state at the room temperature. The reason is to sufficiently impregnate the active metal inside the carrier material as well. The configuration aims to achieve the following advantageous effect. An active metal cation dissolved in the water when the carrier material is placed in the static state is moved (diffused) into the carrier, and the active metal is more uniformly carried. There is no performance problem during a static placement time exceeding 1 hour, but the advantageous effect of uniformly carrying the active metal is saturated. Therefore, the configuration is no longer technically important. In this way, although the static placement time has no upper limit, 1 hour or longer and 2 hours or shorter are substantially sufficient. The obtained impregnation substance is subjected to vacuum drying to remove moisture. Furthermore, the impregnation substance is sintered at 500°C for 3 hours under the dry air circulation. In this manner, a catalyst precursor is obtained. When the auxiliary catalyst is used, a predetermined amount of the active metal raw material and an auxiliary catalyst raw material is dissolved by adding the pure water thereto. Thereafter, the water is added thereto to prepare the impregnation liquid until the active metal raw material and the auxiliary catalyst raw material have the amount of the ADF of the precisely weighed carrier material. The same process may be performed for other configurations.

### <Carrier Material>

As the carrier, a carrier containing at least one type of silica (SiO₂), alumina (Al₂O₃), and zeolite (aluminosilicate) is used. Among these materials, it is preferable to contain silica. With regard to the silica, it is possible to preferably adopt a preparing method such as a sol-gel method in which an alcoholate (alkoxide) compound such as tetraethyl orthosilicate (TEOS, Si(O-C₂H₅)₄) is dissolved in an alcohol compound such as ethyl alcohol, and a condensation polymerization in which an acid or a base is used as the catalyst is performed. With regard to the alumina, in addition to the sol-gel method using the alcoholate compound such as aluminum isopropoxide (Al(O-i-C₃H₇)₃) and alcohol compound such as isopropyl alcohol as in the case of silica, it is possible to preferably adopt a method for sintering and preparing aluminum hydroxide (Al(OH)₃) in dry air. With regard to the zeolite, both natural zeolite and synthetic zeolite can be preferably selected, and as a skeleton structure, an A type, a Y type, a ZSM type, and the like can be preferably selected. The zeolite may be treated with an acid (mineral acid), and may be adjusted to various silica alumina ratios (SiO₂/Al₂O₃ ratio).

### <Active Metal and Auxiliary Catalyst Raw Material>

A water-soluble metal salt can be preferably used as a starting material of the active metal. For example, a fluoride, a chloride, a bromide, a nitrate, a sulfate, an acetate, or the like can be preferably used. Among these materials, the nitrate and the acetate are preferable, and the nitrate is most preferable.

Hereinafter, an example of "a silica carrier, main catalyst cobalt, auxiliary catalyst yttrium" will be described, and a method for preparing the catalyst will be more specifically described. The silica is sintered at 500°C or higher and 520°C or lower for 3 hours or longer under the dry air circulation, thereafter, is cooled at the room temperature, and a predetermined amount is precisely weighed. The cobalt nitrate tetrahydrate (Co(NO₃)₂·4H₂O) and the yttrium nitrate hexahydrate (Y(NO₃)₃·6H₂O) are dissolved by adding the pure water thereto. Furthermore, the pure water is added to have a volume equal to the ADF of a predetermined amount of silica. In this manner, the impregnation liquid is obtained. The sintered silica is immersed in the impregnation liquid, and is placed in a static state at the room temperature for 1 hour. The obtained slurry-like impregnation substance is subjected to vacuum drying at 60°C with a rotary evaporator to remove moisture. Furthermore, the impregnation substance is sintered at 500°C for 3 hours under the dry air circulation. In this manner, the catalyst precursor is obtained.

### <Catalyst Amount>

First, the substance brought into a slurry state is prepared by adding to medium oil 3 in advance the catalyst (before activation) in the amount of 1/2 or less of the weight of the medium oil 3.

Next, a total amount of the medium oil 3 filling the reactor in an initial stage is adjusted by introducing the slurry such that the filling amount of the catalyst reaches 2% by weight or more and 25% by weight or less. The filling amount of the catalyst is more preferably 5% by weight or more and 20% by weight or less, and still more preferably 7% by weight or more and 12% by weight or less.

### <Activation Preparation Process of Catalyst>

As a preparation process of the catalyst for starting the reaction, the catalyst is brought into an active state by performing a series of pretreatment processes of (1) to (4) below on the catalyst.

A primary reduction is to reduce and activate the oxidized catalyst.

A stabilized catalyst is also referred to as an active catalyst precursor, and is a process of stabilizing the catalyst with an inert gas containing a small amount of an oxidizing gas. As the oxidizing gas, oxygen, nitrous oxide (N₂O), or the like can be preferably used, and oxygen is more preferable from a viewpoint of handling.

Catalyst filling is to fill the reaction vessel with the catalyst.

A secondary reduction is to activate the catalyst by heating the catalyst while causing a reducing gas to pass through. As the reducing gas, hydrogen, a hydrogen-steam mixed gas, carbon monoxide, or the like can be preferably used. Hydrogen and a hydrogen-steam mixed gas are more preferable, and hydrogen is still more preferable. An inert gas such as nitrogen and a rare gas may be used in combination with the reducing gas.
(1) Primary reduction: A process of obtaining the active catalyst precursor in such a manner that the catalyst precursor is reduced by circulating hydrogen at a temperature of 400°C or higher and 450°C or lower at a gas hourly space velocity (GHSV) of 300 (v/v) h⁻¹ or higher and 1500 (v/v) h⁻¹ or lower.
(2) Stabilized catalyst: A process of obtaining the stabilized catalyst in such a manner that the inert gas (He, N₂, or the like) containing 0.1% of oxygen is circulated at a temperature of 25°C or higher and 50°C or lower at the gas hourly space velocity (GHSV) of 300 (v/v) h⁻¹ or higher and 600 (v/v) h⁻¹ or lower for 1 hour or longer and 3 hours or shorter.
(3) Catalyst Filling: The stabilized catalyst is added to the medium oil having the hydrocarbon component, and is brought into a slurry state. Next, the hydrogen and the hydrogen-water vapor mixed gas are supplied from a bottom portion of the reactor, and the slurry is introduced into the reactor in a bubble generating state.
(4) Secondary reduction: The hydrogen and the hydrogen-water vapor mixed gas described in (3) are supplied while the pressure inside the reactor is maintained at a pressure in a range of 300 kPa or higher and 600 kPa or lower. The temperature inside the reactor is raised to a range of 150°C or higher and 200°C or lower in the bubble generating state, and is maintained for 1 hour or longer and 3 hours or shorter.

### [Second Embodiment]

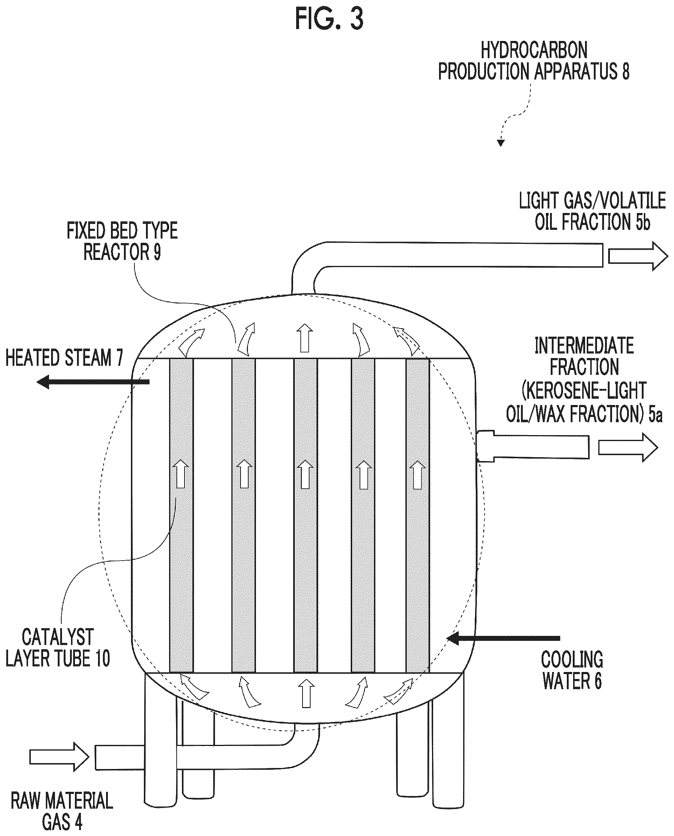

A hydrocarbon production apparatus 8 in a second embodiment includes a fixed bed type reactor 9 shown in Fig. 3. In the hydrocarbon production apparatus 2, unlike the first embodiment, a gas-solid contact reaction is used, and the raw material gas 4 reacts on the catalyst by passing through a catalyst layer tube 10 (catalyst contained in a plurality of tubes having a small diameter). In addition, heat generated by the FT reaction is removed by filling a periphery of the catalyst layer tube 10 with the cooling water 6, and the inside of the reaction vessel is controlled to have a predetermined temperature. The heated cooling water 6 is discharged outward of the apparatus, as the heated steam 7.

In addition, the hydrocarbon production apparatus 8 of the second embodiment using the fixed bed type reactor 9 is the same as the hydrocarbon production apparatus 1 of the first embodiment using the slurry type fluidized bed in that the mixture of the hydrocarbon is produced by the FT reaction.

### Examples

Next, laboratory scale experimental results showing advantageous effects of the present invention will be described below.

### <Conditions>

· Reaction conditions: The pressure = below normal pressure, temperature = 230°C, and reaction time = 7 hours are set.
· Raw material gas: The partial pressure ratio of the hydrogen to the total of (carbon monoxide + carbon dioxide) is set to 1.25.

In addition, the ratios between the carbon monoxide and the carbon dioxide are compared under two conditions that the carbon dioxide is 0% and 20%. However, in order to compare the amounts of the carbon monoxide under a constant condition, when the carbon dioxide is 0%, argon of the inert gas is circulated by 20% of the same amount as when the carbon dioxide is supplied in combination, and the ratios are compared under a condition that the partial pressures of the hydrogen and the carbon monoxide are aligned.
· Catalyst: "yttrium·cobalt/silica carrier" is used. The weight-based carriage amount of cobalt is 10%, the amount of yttrium is 1%, and the weight-based carriage amount of a cobalt main catalyst is 1/10.

### <Measurement Content>

· Product: The produced light gas fraction is collected in a bag, and contents are analyzed by gas chromatography. In addition, a flow rate of the reaction gas and a flow rate of the produced gas are measured.

### <Results>

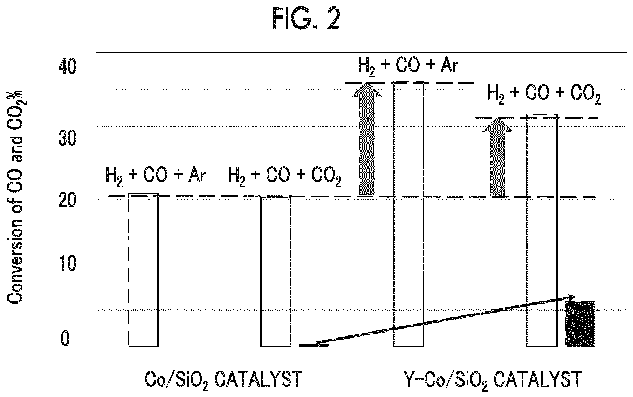

As shown in Fig. 2, the following results are obtained.
· Since the yttrium auxiliary catalyst is used, the carbon conversion rate is improved compared to a case of a yttrium-free catalyst.

The conversion rate of the carbon in the carbon monoxide into the hydrocarbon: In a system of only the carbon monoxide, 20.9 → 36.1% (+15.2%), and in a carbon dioxide coexisting system, 20.3 → 31.5% (+11.2%)

The conversion rate of the carbon in the carbon dioxide into the hydrocarbon: 0.46% → 6.27% (+5.81%) in the carbon dioxide coexisting system
· When the yttrium auxiliary catalyst systems are compared with each other, the conversion rate of the carbon monoxide is decreased by 4.1% due to the coexistence of the carbon dioxide. On the other hand, the conversion rate of the carbon dioxide is improved by +5.8%.

Based on the above-described results, it is possible to confirm the advantageous effect of improving the carbon conversion rate of the carbon dioxide into the hydrocarbon by using the catalyst and the hydrocarbon production apparatus of the present invention. According to these results, under normal pressure, and under a high pressure, the carbon conversion rate of the carbon dioxide into the hydrocarbon is further expected.

The above-described embodiments are merely examples. The catalyst for the FT reaction and the hydrocarbon production apparatus according to the present invention are not limited to the above-described embodiments, and the catalyst or the reaction apparatus according to the above-described embodiments may be modified within the scope which does not change the concept of the appended claims.

### Industrial Applicability

Since the catalyst for the FT reaction and the hydrocarbon production apparatus of the present invention are used, the carbon dioxide can be efficiently converted into the hydrocarbon in the FT reaction of the raw material gas containing the carbon dioxide. In this manner, the present invention contributes to carbon recycling which has been a problem in recent years, and can also be used for coping with environmental problems.

### Reference Signs List

1, 8 Hydrocarbon production apparatus
2 Bubble tower reactor
2a Gas disperser
3 Medium oil
4 Raw material gas
4a Raw material gas bubble
5 Heat exchanger
5a Intermediate fraction (kerosene-light oil/wax fraction)
5b Light gas/volatile oil fraction
6 Cooling water
7 Heated steam
9 Fixed bed type reactor
10 Catalyst layer tube

## Claims

1. A hydrocarbon production apparatus that supplies a raw material gas into a reaction vessel having a reaction catalyst to produce a hydrocarbon by a Fischer-Tropsch synthesis reaction,
wherein the raw material gas contains carbon dioxide, and
the reaction catalyst contains at least one element selected from yttrium, cerium, lanthanum, praseodymium, neodymium, and holmium.

2. The hydrocarbon production apparatus according to claim 1,
wherein the reaction catalyst contains at least one element selected from cobalt, ruthenium, and iron.

3. The hydrocarbon production apparatus according to claim 1 or 2,
wherein a holding amount of the catalyst inside the reaction vessel is 2% by mass or more and 25% by mass or less with respect to a weight of an organic medium inside the reaction vessel.

4. The hydrocarbon production apparatus according to claim 1 or 2,
wherein the raw material gas contains carbon monoxide, and a proportion of the carbon dioxide to a total amount of the carbon monoxide and the carbon dioxide is 1% by volume or higher.

5. The hydrocarbon production apparatus according to claim 1 or 2, further comprising:
a plate-shaped heat exchange unit having irregularities on a surface.

6. A hydrocarbon production method for supplying a raw material gas into a reaction vessel having a reaction catalyst to produce a hydrocarbon by a Fischer-Tropsch synthesis reaction,
wherein the raw material gas contains carbon dioxide, and
the reaction catalyst contains at least one element selected from the group consisting of yttrium, cerium, lanthanum, praseodymium, neodymium, and holmium.

7. A hydrocarbon production method for supplying a raw material gas into a reaction vessel having a reaction catalyst to produce a hydrocarbon by a Fischer-Tropsch synthesis reaction,
wherein the raw material gas contains carbon dioxide, and
the reaction catalyst contains at least one element selected from the group consisting of sodium, potassium, rubidium, and cesium.

8. A hydrocarbon production method for supplying a raw material gas into a reaction vessel having a reaction catalyst to produce a hydrocarbon by a Fischer-Tropsch synthesis reaction,
wherein the raw material gas contains carbon dioxide, and
the reaction catalyst contains copper.

9. A hydrocarbon production method for supplying a raw material gas into a reaction vessel having a reaction catalyst to produce a hydrocarbon by a Fischer-Tropsch synthesis reaction,
wherein the raw material gas contains carbon dioxide, and
the reaction catalyst contains at least one element selected from the group consisting of beryllium, magnesium, calcium, strontium, and barium.
